Europäisches Patentamt

European Patent Office

Office européen des brevets

(11) Publication number: **0 106 729**
**B1**

(12) **EUROPEAN PATENT SPECIFICATION**

(45) Date of publication of patent specification: **09.12.87**

(21) Application number: **83401769.1**

(22) Date of filing: **09.09.83**

(51) Int. Cl.⁴: **B 01 J 37/00,** B 01 J 21/18,
B 01 J 23/40, B 01 J 23/74,
C 07 C 85/11

(54) Method for the preparation of supported catalysts and supported catalysts thus obtained.

(30) Priority: **10.09.82 IT 2319782**

(43) Date of publication of application:
**25.04.84 Bulletin 84/17**

(45) Publication of the grant of the patent:
**09.12.87 Bulletin 87/50**

(84) Designated Contracting States:
**BE CH DE FR GB IT LI NL**

(56) References cited:
**EP-A-0 019 174**
**GB-A-2 024 643**
**US-A-3 736 266**
**US-A-3 974 227**
**US-A-4 158 643**
**US-A-4 239 653**

(73) Proprietor: **MONTEDIPE S.p.A.**
**31, Foro Buonaparte**
**Milan (IT)**

(72) Inventor: **Gubitosa, Giuseppe**
**5 Via della Vecchia**
**Novara (IT)**
Inventor: **Berton, Antonio**
**4 Via Cacciapiatti**
**Novara (IT)**
Inventor: **Pernicone, Nicola**
**7 Via Pansa**
**Novara (IT)**
Inventor: **Vidotto, Graziano**
**47 Via Tre Garofani**
**Padova (IT)**

(74) Representative: **Hirsch, Marc-Roger**
**Cabinet Hirsch 34 rue de Bassano**
**F-75008 Paris (FR)**

Courier Press, Leamington Spa, England.

## Description

The invention concerns a method for the preparation of supported catalysts, particularly suitable for use in the hydrogenation of organic compounds. Furthermore, the invention also relates to the supported catalysts thus obtained.

In general, the amount of supported component (in particular noble metals of Group VIII) is as small as possible, provided that, other conditions remaining the same, the same or better results are obtained than those obtained when greater quantities of catalytic components are used. In other words, the aim is to raise the ratio "carrier/catalytic compound" to a maximum. When the catalyst is used, for instance in the form of a powder dispersed in a liquid reaction medium, the consumption of catalytic component in some instances may be considerable, due not only to the more or less partial solubility of the component in the reaction liquid, but also to the losses during separation of the catalyst from the end product, for instance by filtering or by centrifuging.

Thus, one object of the present invention is to increase the carrier/catalytic compound ratio, while maintaining the parity of the results. Further objects will appear even more evident from reading through the following description.

In its more general form, the invention concerns a method for the preparation of catalysts, particularly suitable for use in the hydrogenation of organic compounds, according to which method a catalytic element, preferably selected from among the VIII group elements and especially from among the group comprising Ni, Pd, Rh and Pt, is supported on the outer surface of an adsorbent or activated carbon, said method being characterized by the fact that:

(a) said carbon, containing an amount of ashes lower than 1%, and preferably 0.6% by weight, is suspended in water;

(b) a liquid, immiscible with water, preferably selected from among the group comprising n-hexane, n-heptane, benzene and toluene, is added to the suspension obtained according to (a), the volume of said immiscible liquid being substantially equal to or lower than the volume of the carriers pores;

(c) an aqueous solution, containing a compound of the catalytic element, is brought into contact with the suspension obtained according to (a) and (b).

Numerous possible forms of embodiment of the present invention are available to the man skilled in the art. Thus, according to one particular convenient form, an activated carbon having a low ash content (that is, containing less than 1%, but preferably less than 0.6% by weight ashes) is suspended in $H_2O$ and is thereafter treated with an aqueous solution of compounds of metals of Group VIII (in such an amount as to have present at the end of from 0.1% to 5% by weight of supported metal), the carbon having been previously put into contact with a solvent immiscible with water, said solvent being present in amounts equal to or smaller than the volume of the pores of the activated carbon.

After the addition of the solvent and before adding the aqueous solution of the catalytic compound, it is advisable to add simultaneously the aqueous solution of an alkaline compound, in such an amount as to reach a pH value (within the suspension) between 7 and 8.5; the same pH after addition of the catalytic compound, must then be brought up to a level equal to or greater than 12.

The preliminary filling up of the pores, in particular those of smaller diameter, with the water-immiscible solvent, hinders the introduction into the inside of the pores of the metal compound which will thus deposit on the outer layer of the granules of the support. If there is treatment to convert such a compound into metal, a catalyst is obtained in which the metal particles are preferably concentrated in said outside layer, in this way more easily accessible for the reactant molecules, thereby avoiding diffusion phenomena of reactants and products inside the porous structure of the catalyst granules, which reactants and products would considerably reduce the catalyst's performance. The present invention may also involve considerable improvements in selectivity, due precisely to the reduction of the mentioned internal diffusion phenomena.

Particularly good results are obtained in the case of Pd-based catalysts (on carbon) used for the hydrogenation of nitro-, nitrous- or nitrillo-organic derivatives, and more particularly of dinitrotoluene (DNT) of toluenediamine (TDA); in this case the simultaneous impregnation of the carbon, treated with an organic solvent, with the aqueous solution of a Pd-compound and of an inorganic base, leads to the formation of more active catalysts than those of the prior art. During the impregnation, the noble metal is deposited on the activated carbon substantially in the form of an extremely dispersed oxide, in concentrations from 0.5% to 5% by weight of Pd. More particularly, the catalysts, in a concentration of 2.5% by weigh of Pd, prepared according to the present process, exert a catalytic activity, expressed as moles of converted organic compound/h/g of metal (for instance, in the hydrogenation of a mixture of 20% of 2,6- dinitrotoluene and 80% of 2,4-dinitrotoluene to the corresponding amines), approximately double with respect to a usual commercial catalyst at 5% of Pd, all the other operative conditions remaining the same. The percentage of ashes is determined by weighing the residue of the calcining in a muffle (e.g. at 700°C during at least 4 hours).

The precursor of the active component of the catalyst, may be a water-soluble Pd-compound, preferably $H_2PdCl_4$, obtained from $PdCl_2$ and from a stoichiometric quantity of HCl. The inorganic base may be a hydroxide of alkaline elements, preferably KOH. The carbon must have a great surface area, between 600 and 1500 $m^2/g$, with a mean pore diameter of 2.5 nm (250 Å) and an apparent density from 0.3 to 0.8 $g/cm^3$. The

catalyst may be dried before use may also be used as such, with considerable liquid content (up to more than 20% by weight), for instance, when a liquid phase hydrogenation is carried out in the presence of a suspended-bed catalyst.

The examples that follow are given for purely illustrative purposes.

## Example 1

The process was started from a commercial carbon produced by LURGI UMWELT u. CHEMOTECHNIK GmbH, of the type Brilonit KE, having a specific surface area of about 800 sq.m/g; this carbon showed a residue in ashes, after calcining in a muffle at 700°C during at least 4 hours, of about 5% by weight. This carbon was then subjected to a purification process with diluted $HNO_3$, according to the method indicated herein-below. 640 g of this commercial carbon were thereupon suspended in 2,5 l of a 10% by weight solution of $HNO_3$ and the ensuing suspension was then maintained under stirring and heated up to 80°C during 4 hours. The carbon was then filtered and repeatedly washed with distilled water (until the filtrate had a pH from 4 to 5) and was not dried before use. 10 grams of the activated carbon thus obtained, having a very low ash content (0.5% by weight), were suspended in 60 $cm^3$ of $H_2O$; the pH of the suspension was 3.05. To this suspension were thereupon admixed, under constant stirring, 5 $cm^3$ of n-heptane, and over a period of 2 hours the pH was stabilized at value 2.1. Thereafter, the pH was brought up to 8 by the addition of 2.6 $cm^3$ of a 0.42 M KOH solution. The suspension was then additioned, over a period of under 1 hour, with 33 $cm^3$ of a $H_2PdCl_4$ solution formed of 0.41 g of $PdCl_2$ and $4.7 \times 10^{-3}$ moles of HCl, while maintaining the pH between 7.5 and 8, by the continuous addition of said KOH solution. At the end of the impregnation, the pH was brought to 12 by further addition of KOH and the suspension was then kept under constant stirring overnight, at room temperature. Successively, the catalyst was filtered and washed with $H_2O$ until a neutral filtrate free from chlorides was obtained. The Pd content, determined by means of atomic absorption, was 2.3% by weight, while the chlorides contents was 0.05% by weight. $60 \times 10^{-3}$ grams of the thus obtained catalyst were loaded into a 200 $cm^{-3}$ glass reactor mounted on a rocking stirrer and connected, through a vacuum-generating system, with a gas-filled burette at constant pressure (substantially atmospheric pressure), and to a compensating funnel, said reactor, burette and funnel being maintained at a thermostatically stabilized temperature of 30°C.

This system was degassed and filled with an inert gas while the catalyst was additioned with 0.5465 g of 2.4-dinitrotoluene dissolved in 15 $cm^3$ of ethylacetate; the inert gas was then substituted with $H_2$, filling the burette with a volume of $H_2$ sufficient for the reaction, whereafter the reactor was subjected, to stirring at 300 strokes/minute. After about 1 hour (the system being kept at 30°C), the solution of DNT was added to the suspension of the catalyst, while stopping stirring for 15 seconds; on resuming the stirring the wasted $H_2$ was determined as a function of time. At the end, when no further absorption of $H_2$ took place, the hydrogenation of DNT was considered to be complete; the gas-chromatography analysis of the reaction solution confirmed this assumption. From the $H_2$ volume it was possible to calculate the reaction rate, expressed as moles of converted DNT/h/g of Pd; the reaction rate, in the case of the catalyst prepared according to example 1, was 3.10 moles/h/g of Pd.

## Example 2

The hydrogenation of example 1 was repeated, while using an Engelhard catalyst prepared according to conventional methods and containing 5% by weight of Pd on an activated carbon carrier, thereby attaining an operational capacity of only 1.6 moles/h/g.

## Example 3

A 1 litre capacity autoclave with a thermostatically stabilized temperature, fitted with a pressure gauge (manometer), a mechanical stirrer and a feeding tank of about 100 $cm^3$, connected through a vacuum-generating system to a $H_2$-containing tank, of known volume and kept at constant temperature, was loaded with an amount of the catalyst prepared according to example 1, equal to $50 \times 10^{-3}$ g. The system was then put under vacuum and filled with an inert gas; to the catalyst were thereupon added 200 $cm^3$ of n-octanol and the tank was then loaded with 12.75 g of DNT and 50 $cm^3$ of n-octanol. The inert gas was then replaced by $H_2$, the autoclave was loaded with $H_2$ under pressure and connected through a pressure reducing valve, with the $H_2$ tank. Both the DNT containing tank as well as the autoclave were heated to a temperature of about 130°C. The mechanical stirring, about 1200 rpm, was already started in the autoclave heating phase. Once the system had stabilized at the desired temperature and pressure (about 130°C and 3 atm. respectively), the DNT solution was added to the catalyst suspension. The volume of $H_2$ was measured with reference to the variation of pressure in the $H_2$-tank; during the tests, both temperature and pressure were maintained rigorously constant.

The conversion rate of DNT (114 moles/h/g of Pd) was determined following the same criteria of example 1 for the test at 30°C and at atmospheric pressure.

## Example 4

Example 3 was repeated, while replacing the catalyst of example 1 by the commercial catalyst of example 2 (Pd=5%); the productivity immediately dropped to 57.7 moles/h/g.

## Examples 5 and 6

Activity tests were carried out within a reactor larger than that used in the previous examples, while using as a reaction medium, a mixture containing 80% of 2.4 TDA and 20% of 2.6 TDA.

The feed contained 75% by weight of the reaction medium mentioned above, and 25% by weight of a mixture containing 80% of 2.4 DNT and 20% of 2.6-DNT. Said feed contained, furthermore, 62 ppm of suspended catalyst. The operative conditions were as follows:

. temperature     130°C
. $H_2$ pressure     3 relative atm.

The two tests were carried out using in one instance the catalyst with 2.5% of Pd, prepared according to the invention (and according to example 1) and in the other instance the catalyst with 5% Pd indicated in example 2. Reaction rates remained the same, and DN-conversions of equal value and equal values for the selectivity to TDA were obtained. This means that the catalyst prepared according to the invention exerts a catalytic activity (moles of converted DNT/h/g), namely, a double amount with respect to the usual catalyst with 5% Pd.

Examples 7 and 8
Example 1 was repeated, apart from the fact that, after impregnation with $H_2PdCl_4$, the pH of the suspension was left at the value 8; the Pd content of 2.25% by weight. The catalytic activity at 30°C and at atmospheric pressures, was 2.4 moles/h/g of Pd, while, at 130°C and 304 kPa (3 atm) it was 103.7 moles/h/g.

Example 9
Example 1 was repeated, apart from the fact that, after having brought the pH to 12, the suspension was heated for about 1 hour to about 90°C; the Pd content was 2.38%, while the catalytic activity, at 30°C and under atmospheric pressure, was 3.15 moles/h/g.

Example 10
10 g of "Brilonit" carbon with a high ash content (5%), as indicated in example 1, were suspended, without pre-treatment with $HNO_3$, in 50 cm$^3$ of $H_2O$. 5 cm$^3$ of n-heptane were then added to this suspension under constant stirring and at pH=9.9. After the suspension pH was stabilized on the value 10, dripping of the $H_2PdCl_4$ solution containing 0.25 g of Pd started. When the pH of the suspension dropped to 7.5, a 0.42 M KOH solution was added in such an amount that the pH of the suspension swinged around value 8. At the end of the addition of the Pd solution, the pH of the catalytic suspension was brought to 12 by a further admixture of KOH solution. The solution was then maintained under stirring overnight. Thereafter, the catalyst was filtered and washed with distilled $H_2O$ until a neutral filtrate free from chlorides was obtained. The Pd content was 2.77% by weight, while the catalyst activity, at 30°C and under atmospheric pressure, was 1.70 moles/h/g.

Example 11
10 g of "Brilonit" carbon, as used in example 10, were suspended in 40 cm$^3$ of distilled $H_2O$, while the pH of the suspension was stabilized on value 3.2 by addition of diluted $HNO_3$. 7 cm$^3$ of n-heptane were successively added and the preparation was then carried on according to example 10. The Pd content was 3% by weight, while the catalytic activity, at 30°C and under atmospheric pressure, was 1.72 moles/h/g.

Example 12
79 g of a carbon with a low ash content, like the one mentioned in example 1, were suspended in 50 cm$^3$ of distilled $H_2O$, and additioned with 7 cm$^3$ of n-hexane. Once the pH of the suspension was stabilized on a value of 2.2, a 0.5 M solution of KOH was added in order to bring the pH value to 7.5. Thereafter a $H_2PdCl_4$ solution containing 0.25 g of Pd at constant pH (8) was added. At the end of the impregnation with $H_2PdCl_4$, the pH was raised to 12 by further addition of KOH. The catalyst was thereupon filtered and washed with $H_2O$ until a neutral filtrate free from chlorides was obtained. The Pd content was 2.82%, while the catalytic activity, at 30°C and under atmospheric pressure, was 4.10 moles/h/g Pd, while at 130°C and under a pressure of 405 kPa (3 atm. (gauge)), it was 130 moles/h/g.

Example 13
Example 12 was repeated, apart from the fact that a smaller amount of $H_2PdCl_4$ was added, as to obtain a catalyst with 1.5% by weight of Pd; the catalytic activity, at 30°C and under atmospheric pressure, was 1.72 moles/h/g.

Example 14
10 g of commercial activated carbon with a low ash content, like the one mentioned in example 1, were suspended in a mixture consisting of 100 cm$^3$ of $H_2O$ and of 8.5 cm$^3$ of benzene. After the pH of the suspension was stabilized on value 2.15, it was brought to 7.5 by the addition of a 0.5 M solution of KOH. Thereafter, to the carbon suspension a $H_2PdCl_4$ solution containing 0.25 g of Pd was added, the pH value being from 7.5 to 8 by the continuous addition of KOH.

On completion of the addition, the pH was brought to 10 by further addition of KOH. The suspension was kept under stirring overnight, and the catalyst was then filtered and washed with $H_2O$ until a neutral filtrate free from chlorides was obtained. The Pd content was 2.60% by weight, while the catalytic activity, at 30°C and under atmospheric pressure, was 3.4 moles/h/g, while at 130°C and under 304 kPa (3 atm) it was 128 moles/h/g.

Example 15
Example 14 was repeated, apart from the fact that the $H_2PdCl_4$ solution contained 0.5 g of Pd. The Pd content of the catalyst was 5% by weight, while the catalytic activity, at 30°C and under atmospheric pressure was 2.14 moles/h/g.

Example 16

Example 11 was repeated, except that the H₂PdCl₄ solution contained 0.1 g Pd; the resulting catalyst contained 1.08% by weight of Pd with a catalytic activity, at 30°C and under atmospheric pressure, of 2.5 moles/h/g.

**Claims**

1. A method for the preparation of catalysts, particularly suitable for use in the hydrogenation of organic compounds, according to which method a catalytic element, preferably selected from among the VIII group elements and especially from the group comprising Ni, Pd, Rh and Pt, is supported on the outer surface of an adsorbent carbon, said method being characterized by the fact that:

(a) said carbon, containing an ash content lower than 1%, and preferably 0.6% by weight, is suspended in water;

(b) a liquid immiscible with water is added to the suspension obtained according to (a), the volume of said immiscible liquid being substantially equal to or lower than the volume of the carrier's pores;

(c) an aqueous solution containing a compound of the catalytic element, is brought into contact with the suspension obtained according to (a) and (b).

2. A method according to claim 1, wherein said immiscible liquid, is selected from the group comprising n-hexane, n-heptane, benzene and toluene.

3. A method according to claim 1, wherein said low-ash carbon is obtained from a carbon having a higher ash content by means of a treatment with an acid, such as, for instance, nitric acid.

4. A method according to claim 1, wherein said low-ash carbon is obtained by means of the degradation of an organic polymer.

5. A method according to claim 1, wherein, after the addition of said immiscible liquid but before the addition of the catalytic solution, the pH of the carrier's suspension is brought to a level between 7 and 8.5.

6. A method according to claim 5, wherein, after the addition of the catalytic solution, the pH is brought to a level equal to or higher than 12.

7. A method for the preparation of a supported catalyst for the hydrogenation of nitro-aromatic compounds, and particularly of nitro- or dinitro-toluenes, according to which method palladium is deposited on the outer surface of a carrier consisting of an adsorbent carbon, said method being characterized by the fact that:

(a) said carbon, containing an ash content lower than 1 but preferably lower than 0.6% by weight is suspended in water;

(b) a liquid immiscible with water, preferably selected from the group comprising n-hexane, n-heptane, benzene and toluene, is added to the suspension obtained according to (a), the volume of said immiscible liquid being substantially equal to or lower than the volume of the carrier's pores;

(c) the pH of the suspension obtained according to (b) is brought to a level between 7 and 8.5;

(d) an aqueous solution, containing a soluble compound of Pd, is brought into contact with the suspension obtained according to (c);

(e) the pH of the suspension obtained according to (d) is brought to a level equal to or higher than 12.

8. A supported catalyst, prepared according to claim 7, wherein Pd is prevailingly distributed on the outer surface of the carrier's granules, said catalyst containing a liquid phase consisting substantially of water and of said immiscible liquid, the amount of liquid phase being at least 20% of the whole weight of the supported catalyst.

9. A catalyst according to claim 8, wherein the amount of Pd is from 0.1 to 5% with respect to the total weight of the catalyst.

10. A process for the hydrogenation of a hydrogenable organic compound, according to which H₂ is brought into contact with said organic compound in the presence of the catalyst according to claims 8 or 9.

11. A process according to claim 10, wherein the catalyst is dried before being used.

12. A process for the manufacture of toluendiamines, by means of a reduction with H₂ of the corresponding dinitro-toluenes, in the presence of the catalyst of claim 9.

13. A process according to claim 12, wherein the catalyst is dried before being used.

14. A process according to claim 12 or 13, wherein the hydrogenation of the dinitro-toluenes is carried out within a hydrogenation solvent consisting of the same toluene-diamines that are obtained from the hydrogenation.

**Patentansprüche**

1. Verfahren zur Herstellung von Katalysatoren, insbesondere von Katalysatoren, die zur Hydrierung organischer Verbindungen geeignet sind, wobei ein vorzugsweise zu der VIII. Gruppe des Periodischen Systems gehöriges katalytisches Element, insbesondere ein zu der Ni, Pd, Rh and Pt umfassenden Gruppe gehöriges Element an der Aussenfläche eines adsorbierenden Kohlenstoffs gehalten wird, dadurch gekennzeichnet,

(a) das man den einen Aschegehalt von weniger 1%, vorzugsweise 0,6% aufweisenden Kohlenstoff in Wasser suspendiert;

(b) dass man der im Verfahrensschritt (a) erzielten Suspension eine mit Wasser nicht mischbare Flüssigkeit beimengt, wobei das Volumen der nicht mischbaren Flüssigkeit im Wesentlichen gleich dem Volumen der Poren des Trägers oder kleiner als dieses Volumen ist;

(c) dass man mit der in den Verfahrensschritten (a) und (b) erzielten Suspension eine eine Verbindung des katalytischen Elements enthaltende wässerige Lösung in Berührung bringt.

2. Verfahren nach Anspruch 1, bei dem die nicht mischbare Flüssigkeit zu der n-Hexan, n-Heptan, Benzol und Toluol umfassenden Gruppe gehört.

3. Verfahren nach Anspruch 1, bei dem der Kohlenstoff mit niedrigem Aschegehalt aus einem einen höheren Aschegehalt besitzenden Kohlenstoff durch eine Behandlung mit Säure, wie z.B. Salpetersäure hergestellt wird.

4. Verfahren nach Anspruch 1, bei dem der Kohlenstoff mit niedrigem Aschegehalt durch den Abbau eines organischen Polymers hergestellt wird.

5. Verfahren nach Anspruch 1, bei dem man das pH der Trägersuspension nach der Beimengung der nicht mischbaren Flüssigkeit, jedoch vor der Beimengung der katalytischen Lösung auf einen Wert von 7 bis 8,5 einstellt.

6. Verfahren nach Anspruch 5, bei dem man nach der Beimengung der katalytischen Lösung das pH auf einen Wert von wenigstens 12 einstellt.

7. Verfahren zur herstellung eines auf einem Träger befindlichen Katalysators zur Hydrierung von Nitroverbindungen oder nitroaromatischen Verbindungen, insbesondere Nitrotoluol oder Dinitrotoluol, bei dem man Palladium auf die äussere Oberfläche eines aus adsorbierendem Kohlenstoff bestehenden Trägers aufbringt, dadurch gekennzeichnet,

(a) dass man den einen Aschegehalt von weniger als 1%, vorzugsweise weniger als 0,6% aufweisenden Kohlenstoff in Wasser suspendiert;

(b) dass man der im Verfahrensschritt (a) erzielten Suspension eine zur n-Heptan, n-Hexan, Benzol, und Toluol umfassenden Gruppe gehörige, mit Wasser nicht mischbare Flüssigkeit beimengt, wobei das Volumen der nicht mischbaren Flüssigkeit im Wesentlichen dem Volumen der Trägerporen gleich oder geringer als dieses Volumen ist;

(c) dass man das pH der im Verfahrensschritt erzielten Suspension auf einen Wert von 7 bis 8,5 einstellt;

(d) dass man eine eine lösliche Pd-Verbindung enthaltende wässerige Lösung mit der im Verfahrensschritt (c) erzielten Suspension in Berührung bringt;

(e) dass man das pH der im Verfahrensschritt (d) erzielten Suspension auf einen Wert von wenigstens 12 einstellt.

8. Auf einem Träger befindlicher Katalysator, der vermittels des Verfahrens nach Anspruch 7 hergestellt ist, bei dem das Pd vorwiegend auf der Aussenfläche der Trägerteilchen verteilt ist, wobei der Katalysator eine wesentlich aus Wasser und der nicht mischbaren Flüssigkeit bestehende flüssige Phase enthält, die wenigstens 20% des Gesamtegewichts des auf dem Träger befindlichen Katalysators darstellt.

9. Katalysator nach Anspruch 8, bei dem das Pd in einer Menge von 0,1 bis 5% in bezug auf das Gesamtgewicht des Katalysators vorliegt.

10. Verfahren zum Hydrieren einer hydrierbaren organischen Verbindung, bei welchem H$_2$ mit der organischen Verbindung in Anwesenheit des Katalysators nach Anspruch 8 oder 9 in Berührung gebracht wird.

11. Verfahren nach Anspruch 10, bei dem der Katalysator vor seiner Verwendung getrocknet wird.

12. Verfahren zur Herstellung von Toluoldiamin, bei dem man ein entsprechendes Dinitrotoluol in Anwesenheit des Katalysators nach Anspruch 9 mit H$_2$ reagieren lässt.

13. Verfahren nach Anspruch 12, bei dem der Katalysator vor seiner Verwendung getrocknet wird.

14. Verfahren nach Anspruch 12 oder 13, bei dem die Hydrierung von Dinitrotoluol in einem Hydrier=Lösungsmittel erfolgt, das aus dem durch die Hydrierung erzeugten Toluoldiamin besteht.

**Revendications**

1. Procédé de préparation de catalyseurs, utilisables notamment pour l'hydrogénation de composés organiques, dans lequel un élément catalytique, choisi de préférence parmi les éléments du groupe VIII, et plus particulièrement parmi les éléments formant le groupe qui comprend Ni, Pd, Rh et Pt, est supporté sur la surface extérieure d'un carbone adsorbant, ledit procédé étant caractérisé par le fait que:

(a) le carbone, qui présente une teneur en cendres inférieure à 1% et de préférence inférieure à 0,6% en poids, est suspendu dans l'eau;

(b) un liquide non miscible à l'eau est ajouté à la suspension obtenu dans l'étape (a), le volume dudit liquide non miscible étant sensiblement égal ou inférieur au volume des pores du support;

(c) une solution aqueuse renfermant un composé de l'élément catalytique est mis en contact avec la suspension obtenue dans les étapes (a) et (b).

2. Procédé selon la revendication 1, dans lequel ledit liquide non miscible est choisi parmi les liquides du groupe constitué par: n-hexane, n-heptane, benzène et toluène.

3. Procédé selon la revendication 1, dans lequel ledit carbone à faible teneur en cendres est obtenu à partir d'un carbone présentant une teneur plus élevée en cendres, par un traitement à l'aide d'un acide, tel que l'acide nitrique.

4. Procédé selon la revendication 1, dans lequel ledit carbone à faible teneur en cendres est obtenu par la dégradation d'un polymère organique.

5. Procédé selon la revendication 1, dans lequel on ajuste le pH de la suspension du support à une valeur comprise entre 7 et 8,5, après l'addition dudit liquide non miscible, mais avant l'addition de la solution catalytique.

6. Procédé selon la revendication 5, dans lequel on ajuste le pH à une valeur égale ou supérieure à 12, après l'addition de la solution catalytique.

7. Procédé de préparation d'un catalyseur sur support pour l'hydrogénation de composés nitro-aromatiques, et plus particulièrement des nitrotoluènes ou dinitro-toluènes, dans lequel on dépose du palladium sur la surface extérieure d'un support formé d'un carbone adsorbant, ledit procédé étant caractérisé par le fait que:

(a) l'on suspend dans de l'eau ledit carbone, dont la teneur en cendres est inférieure à 1, et de préférence inférieure à 0,6% en poids;

(b) on ajoute à la suspension dans l'étape (a) un liquide non miscible à l'eau choisi parmi les composants du groupe comprenant: n-hexane, n-heptane, benzène et toluène, le volume dudit liquide non miscible étant sensiblement égal ou inférieure au volume des pores du support.

(c) on ajuste le pH de la suspension obtenue dans l'étape (b) à une valeur comprise entre 7 et 8,5:

(d) on met en contact avec la suspension obtenue dans l'étape (c) une solution aqueuse renfermant un composé soluble de Pd;

(e) on ajuste le pH de la suspension obtenue dans l'étape (d) à une valeur égale ou supérieure à 12.

8. Catalyseur sur support, préparé selon le procédé défini dans la revendication 7, dans lequel du Pd est réparti, de manière prépondérante, sur la surface extérieure des granules dudit support, ledit catalyseur renfermant une phase liquide constituée essentiellement par de l'eau et ledit liquide non miscible, cependant que la proportion de la phase liquide représente au moins 20% du poids total dudit catalyseur sur support.

9. Catalyseur selon la revendication 8, dans lequel la proportion de Pd est de 0,1 à 5% par rapport au poids total du catalyseur.

10 Procédé d'hydrogénation d'un composé organique hydrogénable, dans lequel $H_2$ est mis en contact avec ledit composé organique en présence du catalyseur selon la revendication 8 ou 9.

11. Procédé selon la revendication 10, dans lequel ledit catalyseur est séché avant son utilisation.

12. Procédé de préparation de toluène-diamines par réduction, à l'aide de $H_2$ des dinitro-toluènes correspondants, en présence du catalyseur selon la revendication 9.

13. Procédé selon la revendication 12, dans lequel ledit catalyseur est séché avant son utilisation.

14. Procédé selon la revendication 12 ou 13, dans lequel on effectue l'hydrogénation des dinitro-toluènes dans un solvant d'hydrogénation constitué par les diamines de toluène qui sont obtenues par ladite hydrogénation.